# EUROPEAN PATENT SPECIFICATION

(11) **EP 0 381 345 B1**
(45) Date of publication and mention of the grant of the patent: **13.07.1994**
(21) Application number: 90300622.9
(22) Date of filing: 22.01.1990
(51) Int. Cl.: A61K 9/00, A61K 37/34, A61K 47/38

(54) **Aqueous liquid desmopressin-CMC pharmaceutical composition**
Wässerige Desmopressin-CMC enthaltende Arzneizubereitung
Composition aqueuse à base de desmopressine-CMC

(30) Priority: 30.01.1989 US 304862; 14.11.1989 US 435753
(43) Date of publication of application: 08.08.1990
(73) Proprietor: CORINT, LTD., CH-1211 Geneve 1 (CH)
(72) Inventor: Schrell, Uwe, D-8520 Erlangen (DE)
(74) Representative: Crisp, David Norman

(56) References cited:
- WO-A-88/05661

## Description

The present invention relates to a method for the preparation of a pharmaceutical composition containing biologically-active desmopressin ("dDAVP", a vasopressin-based polypeptide) in combination with carboxymethyl cellulose ("CMC"). More particularly, the invention is generally concerned with a method for the preparation of a non-orally administered, liquid pharmaceutical composition, which comprises an aqueous solution of dDAVP and water-soluble CMC, and which exhibits prolonged activity and increased bioavailability of dDAVP.

It has long been recognized that natural vasopressin has biological effects, such as antidiuretic activity and vasoconstriction of visceral blood flow. Its utility, however, is limited by its relatively short half-life in the blood stream, as well as its well known general circulatory systemic pressor effect, and cardiac toxicity.

A variety of analogs of vasopressin have been synthesisized in an effort to modify the properties of vasopressin and provide products having increased pharmaceutical utility. For example, vasopressin has been modified by deamination of cysteine at the 1 position and replacement of arginine by its D-isomer at the 8 position to yield desmopressin. Desmopressin, which is immune to enzymatic cleavage of the 1-2 and 8-9 carbon-nitrogen bonds, exhibits prolonged antidiuretic activity with low pressor activity. See U.S. Patent No. 3,497,491. In further modifications, the disulfide bridge has been replaced with a "monocarba" linkage (-CH₂S- or -SCH₂-) or a dicarba (-CH₂-CH₂-) linkage.

These vasopressin-based polypeptides have been administered by a variety of routes and in combination with a variety of pharmaceutical carriers and additives. For example, desmopressin has been administered intranasally, subcutaneously, intravenously, and intramuscularly in physiological saline at pH 4.

Various attempts have also been made to effectively orally administer vasopressin-based polypeptides, such as desmopressin. It has traditionally been accepted that such peptides are decomposed in the gastrointestinal tract with little nonapeptide absorption taking place. Nonetheless, dDAVP has been incorporated in gelatin-based sub-lingual lozenges. (A. Grossman et al. "Two new modes of desmopressin (dDAVP) administration", British Medical Journal, May 17, 1980, 1215.) Additionally, dDAVP has been formulated into an orally administered composition in the form of a tablet with various fillers and inert constituents, including crosslinked CMC. (International Patent Application No. PCT/US84/01860, H. Hagstam et al.), but the effective unit dosage appears to be ten times the effective intranasal dosage conventionally required, and no difference in duration of antidiuretic action was reported or claimed.

Hagstam et al. disclose that such compositions are capable of dissolving and being absorbed in the gastrointestinal tract. However, this absorption apparently results from the selective use of the cleavage-resistant analog dDAVP; there is no indication that cross-linked CMC functions as other than an inert constituent. Indeed, in the example given, the crosslinked CMC was only a minor component of a mixture of several excipients, and was present at a rate of only 2 to 4 parts per 100 parts of desmopressin.

U.S. Patent No. 3,454,549 discloses still another vasopressin analog, 1-desamino-[8-L-Arg]-vasopressin, and states that it can be used in the form of its free base, or as a salt of an organic or inorganic acid, on an acid-radical containing polymer, with CMC being mentioned as an illustrative acid polymer. There is no recognition, however, that any acid salt, much less the CMC salt, has any effect on the activity of the vasopressin analog.

WO-A-8805661 describes pharmaceutical compositions containing biologically active vasopressin-based polypeptides, including desmopressin, in combination with CMC. The compositions, which may be in injectible form, exhibit prolonged activity and increased bioavailability of the polypeptide.

The present invention provides a method for the preparation of a sterile aqueous liquid pharmaceutical composition which comprises an aqueous solvent for the ingredients of said liquid pharmaceutical composition, water-soluble carboxymethyl cellulose (CMC), and desmopressin, wherein the desmopressin is combined with the water-soluble carboxymethyl cellulose in an amount sufficient to provide enhanced and prolonged activity of the desmopressin, wherein the weight ratio of CMC to polypeptide is from 100:1 to 200:1, said method comprising separately heat sterilizing the CMC and ultra filter sterilizing the desmopressin and combining the separately sterilized components.

In another aspect, the invention provides for the use in the method of the present invention of desmopressin in association with water-soluble carboxymethyl cellulose (CMC) for the preparation of a sterile medicament for treating diabetes insipidus, enuresis, incontinence, Factor VIII deficiency and/or sickle cell anemia, wherein the sterile pharmaceutical composition is prepared by separately heat sterilizing the CMC and ultra filter sterilizing the desmopressin and combining the separately sterilized components.

The invention also provides for the use in the method of the present invention of desmopressin in association with water-soluble carboxymethyl cellulose (CMC) for the preparation of a medicament for treating Factor VIII deficiency.

The composition has enhanced physiological activity and a long effective lifetime as compared with the dDAVP alone.

Various preferred features and embodiments of the invention are described below. It is to be understood, however, that it is not intended to limit the invention to the specific forms disclosed.

In general, the present invention is predicated on the discovery that a dDAVP-containing pharmaceutical composition can be provided, which has enhanced pharmacological activity and increased duration of action, by combining dDAVP with water-soluble CMC. dDAVP, the vasopressin-based polypeptide employed in an aqueous solution in the invention, may be represented by the following formula: 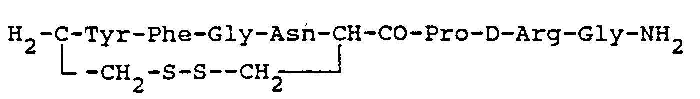
It is believed that CMC is able to prolong and enhance the activity of dDAVP because it is able to form a complex with dDAVP. dDAVP has a high density of amino groups which are available for complexing via non-covalent association with the carboxyl groups of carboxymethylcellulose, i.e. the omega positions of 4 and 5, the guanidino omega side-chain group of Arg at position 8, and the C-terminal (position 9) amide.

Previously, CMC has been used as an inert and safe "filler" in tablet formulations, while the sodium salt of CMC has been given orally as a weak antacid. CMC has also been safely used in injectable formulations. However, the present invention is premised on the fact that CMC can be noncovalently complexed or otherwise combined with dDAVP in aqueous solution to provide a liquid pharmaceutical composition showing increased bioavailability and prolonged effect of dDAVP.

The specific form of CMC is not critical, provided it is water-soluble. The amount of CMC should be sufficient to impart improved dDAVP activity, as reflected by increased activity and/or increased half-life. As a general rule, however, the weight ratio of CMC to dDAVP should be at least 100:1. Ratios of from 100:1 to 200:1 are preferred, with a ratio of about 100:1 being especially preferred. In particular, if the composition is used in the form of an injectable solution, higher amounts of CMC should be avoided. Otherwise, the viscosity of the resulting solution will be too high.

On the other hand, a minimum amount of CMC must be complexed with dDAVP in order to achieve the advantages of the invention. The degree to which dDAVP is complexed at any pH will depend to some extent on the isoelectric point of dDAVP, and the amount and type of other ionic and polar molecules which are present to compete with the polypeptide and CMC.

Although the use of the pharmaceutical compositions prepared by the invention, dosage and route of administration will ultimately depend upon the patient's unique conditions, and the judgment of his attending physician, they can generally be administered to the patient being treated at dosages and by routes calculated to deliver effective amounts of dDAVP to the site of action. For example, they may be administered by intravenous, subcutaneous, or intramuscular injection, or they may be administered intranasally in the form of nose drops or spray.

With these routes of administration, the composition prepared by this invention can be administered as a solution in a suitable aqueous liquid medium, such as water, saline, isotonic aqueous solution. The medium may contain various pharmaceutically acceptable fillers and additives, such as anticlotting agents, dispersing agents, acidifying agents and the like. A preferred medium is physiological saline solution. If the formulation is for multiple use, it is also desirable to include in the dispersion or solution a small amount of a physiologically acceptable bacteriostat, e.g., chlorobutanol, to minimize bacterial contamination. This is especially useful in the intranasal preparation.

In addition, in preparing the compositions in accordance with the invention, separately sterilized solutions of dDAVP and CMC should be prepared and then mixed together. The CMC solution is heat sterilized, preferably below 120°C, and more preferably between about 105°C to about 120°C and with optional stirring, followed by centrifugation as required to remove any undissolved particles. Since heat treatment might lead to inactivation of dDAVP, the dDAVP solution is separately sterilized by ultrafiltration. In general, the CMC solutions employed in the present invention will be too viscous for ultra-membrane filtration.

When preparing a sterile formulation pursuant to the foregoing procedure, it is convenient to prepare and combine equal volumes of double strength solutions of CMC and dDAVP, which have been separately sterilized, however other concentration and amounts can be used as appropriate to yield the pharmaceutical composition.

The concentration of the dDAVP in the solution is not narrowly critical and will depend upon the pH, the intended mode of administration, and dosage. In general, solutions intended for intranasal applications may contain higher concentrations than solutions intended to be administered by injection, but dimerisation and peptide stability are usually the decisive factors here.

The dosage of the composition which is administered will depend greatly upon the specific action(s) of the polypeptide, the level(s) of such action(s), the effect intended, and the mode of administration. For example, when administered in the form of nose drops, the applied dosage will be about 10 times the applied dose administered by intravenous routes.

However, as shown in the Examples, because the pharmaceutical composition prepared by the invention has enhanced bioavailability and prolonged effect, the effective dosage may be significantly decreased for all modes of administration pursuant to the invention.

The invention will be further described by reference to the following detailed examples, which are not intended to be limiting, but rather, illustrative of some approaches taken.

### EXAMPLE 1

### Subcutaneous Administration of dDAVP to Rats

An acid physiological saline solution (pH 4) containing 10 »g/ml of Desmopressin (dDAVP) (manufactured by Ferring AB) and chlorobutanol was prepared and divided into two aliquots. One of the aliquots was set aside, while the other aliquot was mixed with sufficient Na-CMC (Tylopur C30, manufactured by Kalle) to obtain a CMC concentration of 2 mg/ml. Thus, the CMC:dDAVP weight ratio of the CMC-containing aliquot was 200:1, at a pH of 4.

Male rats having a body weight of 180-200 g were anesthetized with Methohexital-Na (Brevimytal, manufactured by Lilly), and then surgically prepared by inserting a tracheal cannula into a carotid artery in order to draw blood samples (0.5 ml/sample) into heparinised tuberculin syringes, and a polyethylene cannula into a jugular vein to rehydrate and replace blood volumes lost during sampling, by injecting equal volumes of iso-oncotic dextran in saline. Both cannulae were inserted in a central direction. Zero-time samples were withdrawn to serve as a baseline.

dDAVP and dDAVP-CMC solutions were then subcutaneously injected into the rats in a volume dose of 0.1 ml/100g body weight. Blood samples were withdrawn at 5, 10, 20, and 40 minutes after injection. These times were chosen because the half-life (T_{1/2}) of dDVAP in man after intravenous (i.v.) injection has already been determined to be 17-18 minutes. (K.E. Andersson et al., Acta Endocrinol, 69:640, 1972), as opposed to about 3 minutes for the natural vasopressins. (V. Pliska et al., Experientia. 29:767, 1973).

The heparinised samples were stirred and centrifuged. Then, the supernatant plasma was pipetted off and stored in separate plastic tubes at -70°C until analyzed.

The plasma levels of dDAVP in the withdrawn samples were detected by radio-immune assay ("RIA"), using a high affinity rabbit antibody prepared by Dr. M. Sofroniew of the Department of Anatomy at the University of Munchen and the results presented in Table 1 below. A commercial goat-anti-rabbit plasma was used to precipitate Ag-Ab complexes, while ₁₂₅I was used to label the 2-Tyr of the dDAVP. Even though the Ab used was Arg-vasopressin specific, it showed sufficient cross-reactivity with other vasopressins, including dDAVP, to allow detection of plasma levels of dDAVP down to 7 pg/ml.

Because of the small plasma samples involved, no extraction methods were applied to the plasma samples. For this reason, higher than usual 0-time levels were recorded and in all calculations the 0-time level was subtracted from all levels recorded after drug injection.

**Table 1**

| Plasma Peak Time (minutes) | dDAVP | dDAVP-CMC |
|---|---|---|
| Means | 8.8* | 21.5* |
| ±S.D. | 1.3 | 6.6 |
| ±SEM | 0.9 | 2.7 |
| ±95CL | 1.7 | 5.3 |

| Plasma (T_{1/2}) (minutes) | | |
|---|---|---|
| Means | 20.5* | 58.8* |
| ±S.D. | 0.5 | 10.9 |
| ±SEM | 0.35 | 4.9 |
| ±95CL | 0.7 | 9.6 |

| Plasma peak concentrations (pg/ml) | | |
|---|---|---|
| Means | 27.0* | 82.0* |
| ±S.D. | 1.5 | 38.7 |
| ±SEM | 1.1 | 17.3 |
| ±95CL | 2.1 | 34.0 |
| All means pairs followed by * indicate a statistically significant difference P < 0.05. | | |

In the above Table (and below), "S.D." means "standard deviation", "SEM" means "standard error of the mean", and "95CL" means "95% confidence limit".

The reported data clearly show that increased plasma concentration half-lives were obtained using the dDAVP-CMC compositions prepared by the invention. In connection with the above, it is noted that the reported T_{1/2} values for dDAVP alone were generally in agreement with the previously reported values obtained with i.v. injection in man. The larger mean value reported here undoubtedly resulted because absorption is relatively slower after s.c. injection.

Additionally, the reported plasma T_{1/2} values should not be confused with a biological response T_{1/2}, which would be much longer in all cases (c.f. Example 2). However, the increased T_{1/2} value obtained using a dDAVP/CMC combination indicates that dDAVP activity would be even more prolonged in vivo.

The above data also show that a peak concentration was reached after a significantly longer time when the dDAVP/CMC combination was used. Again, both mean peak values were greater than would be obtained after i.v. injection.

Perhaps the most striking difference associated with the use of CMC was the significantly higher peak plasma concentrations achieved with dDAVP-CMC vs. dDAVP alone. Although no definitive explanation is offered at this time, it is possible that slower release of dDAVP from the dDAVP-CMC complex decreases the rate of either enzymatic cleavage or disappearance of dDAVP into other receptor fields than the target organ. In other words, combining dDAVP with CMC appears to increase the bioavailability of the active peptide.

### EXAMPLE 2

### Antidiuretic Response to Intranasal Administration of dDAVP in Man

Normal test subjects were loaded with 1.5% body weight of water under constantly maintained conditions, with urine being collected every 30 minutes, as described by J.H. Cort et al., Kidney International. 8:292, 1975, and then they were administered dDAVP intranasally, using a standard 0.1 mg/ml formulation obtained from Ferring AB of Malmo. A portion of the dDAVP was set aside, while Na-CMC was added to another portion to form a solution having a CMC:dDAVP weight ratio of 100:1.

The dDAVP and dDAVP-CMC solutions were intranasally administered to each subject in doses of 2.5 »g/nostril or 5 »g/patient. The results are given in Table 2.

**Table 2**

| Peak % Inhibition of Diuretic Urine Flow | dDAVP | dDAVP-CMC |
|---|---|---|
| Means | -76.7* | -97.4* |
| ±S.D. | 3.7 | 1.9 |
| ±SEM | 1.5 | 1.1 |
| ±95 CL | 3.0 | 2.2 |

| T_{1/2} of Antidiuretic Response (hours) | | |
|---|---|---|
| Means | 4.92* | 11.0* |
| ±S.D. | 1.30 | 4.08 |
| ±SEM | 0.53 | 2.36 |
| ±95 CL | 1.04 | 4.65 |

There were no side-effects or discomfort associated with the intranasal use of dDAVP-CMC but there was a 15-20 minute delay in the onset of the antidiuretic response. Increased bioavailability induced by the CMC complexing was evidenced both by the significant increase in peak antidiuretic response and the approximate doubling of the biological (antidiuretic) T_{1/2}. This doubling of the duration half-time (total time would be expected to show an even greater increase in prolongation) would indicate that: a) the effective average dose of peptide in dDAVP-CMC could be halved from 10 »g to 5 »g/adult Diabetes insipidus patient and b) this halved dose could be given once daily instead of twice daily (b.i.d).

### EXAMPLE 3

### Preparation of Sterile dDAVP-CMC Solution

A sterile solution of 0.1mg dDAVP and 10.0 mg CMC in physiological saline at pH 4 is prepared as follows.

A solution of 0.2mg/ml dDAVP is prepared in physiological saline (1N HCl is added to adjust the solution pH to 4), and then ultrafiltered at room temperature under sterile conditions. A separate solution of CMC is prepared by thoroughly mixing 20 mg/ml CMC in physiological saline at pH 4 and 30°C for 30-60 minutes. The CMC solution is separately sterilized by heating to 105°C for 1 hour, followed by centrifugation at 5000 g to remove any undissolved particles. Then, equal volumes of the separately sterilized double strength solutions are combined to provide the aqueous pharmaceutical composition prepared by the invention.

## Claims

1. A method for the preparation of a sterile aqueous liquid pharmaceutical composition which comprises an aqueous solvent for the ingredients of said liquid pharmaceutical composition, water-soluble carboxymethyl cellulose (CMC), and desmopressin, wherein the desmopressin is combined with the water-soluble carboxymethyl cellulose in an amount sufficient to provide enhanced and prolonged activity of the desmopressin, wherein the weight ratio of CMC to polypeptide is from 100:1 to 200:1, said method comprising separately heat sterilizing the CMC and ultra filter sterilizing the desmopressin and combining the separately sterilized components.

2. A method according to claim 1, wherein the weight ratio of carboxymethyl cellulose to polypeptide is 100:1.

3. A method according to claim 1 or claim 2, further comprising a bacterial growth inhibitor.

4. A method according to any preceding claim wherein the CMC is sterilized at a temperature below 120°C.

5. A method according to claim 4 wherein the CMC is sterilized at a temperature in the range of 105° to 120°C.

6. A method according to any preceding claim wherein the CMC is sterilized at a temperature of 105°C.

7. A method according to any preceding claim wherein the sterilized CMC component is centrifuged to remove undissolved particles.

8. Use in the method of any one of claims 1 to 7 of desmopressin in association with water-soluble carboxymethyl cellulose (CMC) for the preparation of a sterile medicament for treating diabetes insipidus, enuresis, incontinence, Factor VIII deficiency and/or sickle cell anemia, wherein the sterile pharmaceutical composition is prepared by separately heat sterilizing the CMC and ultra filter sterilizing the desmopressin and combining the separately sterilized components.

9. Use according to claim 8 wherein the medicament comprises an aqueous solution of desmopressin and water-soluble CMC.

10. Use according to either of claims 8 and 9 wherein the CMC is sterilized at a temperature below 120°C.

11. Use according to claim 10 wherein the CMC is sterilized at a temperature in the range 105° to 120°C.

12. Use according to any one of claims 8 to 11 wherein the CMC is sterilized at a temperature of 105°C.

13. Use according to any one of claims 8 to 12 wherein the sterilized CMC component is centrifuged to remove undissolved particles.

14. Use according to claim 8 of desmopressin in association with water-soluble carboxymethyl cellulose (CMC) for the preparation of a medicament for treating Factor VIII deficiency.

15. Use according to claim 14 wherein the medicament comprises an aqueous solution of desmopressin and water-soluble CMC.

## Patentansprüche

1. Verfahren zur Herstellung einer sterilen wäßrigen flüssigen pharmazeutischen Zusammensetzung, die ein wäßriges Lösemittel für die Bestandteile dieser flüssigen pharmazeutischen Zusammensetzung, wasserlösliche Carboxymethylcellulose (CMC) und Desmopressin umfaßt, unter Vereinigung des Desmopressins mit der wasser-löslichen Carboxymethylcellulose in einer ausreichenden Menge, um verbesserte und verlängerte Aktivität des Desmopressins zu bekommen, wobei das Gewichtsverhältnis von CMC zu Polypeptid 100 : 1 bis 200 : 1 beträgt, indem man getrennt die CMC hitzesterilisiert und das Desmopressin ultrafiltersterilisiert und die getrennt sterilisierten Bestandteile miteinander vereinigt.

2. Verfahren nach Anspruch 1, bei dem das Gewichtsverhältnis von Carboxymethylcellulose zu Polypeptid 100 : 1 ist.

3. Verfahren nach Anspruch 1 oder Anspruch 2, das zusätzlich einen Bakterienwachstumsinhibitor umfaßt.

4. Verfahren nach einem der vorausgehenden Ansprüche, bei dem die CMC bei einer Temperatur unterhalb 120 °C sterilisiert wird.

5. Verfahren nach Anspruch 4, bei dem die CMC bei einer Temperatur im Bereich von 105 bis 120 °C sterilisiert wird.

6. Verfahren nach einem der vorausgehenden Ansprüche, bei dem die CMC bei einer Temperatur von 105 °C sterilisiert wird.

7. Verfahren nach einem der vorausgehenden Ansprüche, bei dem die sterilisierte CMC-Komponente zentrifugiert wird, um ungelöste Teilchen zu entfernen.

8. Verwendung von Desmopressin in Verbindung mit wasserlöslicher Carboxymethylcellulose (CMC) in dem Verfahren nach einem der Ansprüche 1 bis 7 für die Herstellung eines sterilen Arzneimittels zur Behandlung von Diabetes insipidus, Enurese, Inkontinenz, Mangel an Faktor VIII und/oder krankhafter Zellenanämie, wobei man die sterile pharmazeutische Zusammensetzung herstellt, indem man getrennt die CMC hitzesterilisiert und das Desmopressin ultrafiltersterilisiert und die getrennt sterilisierten Bestandteile miteinaner vereinigt.

9. Verwendung nach Anspruch 8, bei der das Arzneimittel eine wäßrige Lösung von Desmopressin und wasserlöslicher CMC umfaßt.

10. Verwendung nach einem der Ansprüche 8 und 9, bei der die CMC bei einer Temperatur unterhalb 120 °C sterilisiert wird.

11. Verwendung nach Anspruch 10, bei der die CMC bei einer Temperatur im Bereich von 105 bis 120 °C sterilisiert wird.

12. Verwendung nach einem der Ansprüche 8 bis 11, bei der die CMC bei einer Temperatur von 105 °C sterilisiert wird.

13. Verwendung nach einem der Ansprüche 8 bis 12, bei der die CMC-Komponente zentrifugiert wird, um ungelöste Teilchen zu entfernen.

14. Verwendung von Desmopressin in Verbindung mit wasserlöslicher Carboxymethylcellulose (CMC) nach Anspruch 8 für die Herstellung eines Arzneimittels zur Behandlung von Mangel an Faktor VIII.

15. Verwendung nach Anspruch 14, bei der das Arzneimittel eine wäßrige Lösung von Desmopressin und wasserlöslicher CMC umfaßt.

## Revendications

1. Procédé de préparation d'une composition pharmaceutique liquide aqueuse stérile, comprenant un solvant aqueux pour les ingrédients de ladite composition pharmaceutique liquide, de la carboxyméthylcellulose (CMC) soluble dans l'eau, et de la desmopressine, dans lequel la desmopressine est combinée avec la carboxyméthylcellulose soluble dans l'eau en une quantité suffisante pour fournir une activité augmentée et prolongée de la desmopressine, le rapport pondéral de la CMC au polypeptide valant de 100:1 à 200:1, ledit procédé comprenant les étapes séparées consistant à stériliser à la chaleur la CMC, stériliser par ultrafiltration la desmopressine et combiner les composants stérilisés séparément.

2. Procédé selon la revendication 1, où le rapport pondéral de la carboxyméthylcellulose au polypeptide est de 100:1.

3. Procédé selon la revendication 1 ou 2, comprenant, en outre, un inhibiteur de croissance bactérienne.

4. Procédé selon l'une quelconque des revendications précédentes, où la CMC est stérilisée à une température inférieure à 120°C.

5. Procédé selon la revendication 4, où la CMC est stérilisée à une température comprise entre 105°C et 120°C.

6. Procédé selon l'une quelconque des revendications précédentes, où la CMC est stérilisée à la température de 105°C.

7. Procédé selon l'une quelconque des revendications précédentes, où on centrifuge le composant CMC stérilisée pour enlever des particules non dissoutes.

8. Utilisation de la desmopressine dans le procédé selon l'une quelconque des revendications 1 à 7, en association avec de la carboxyméthylcellulose (CMC) soluble dans l'eau, pour la préparation d'un médicament stérile pour le traitement du diabète insipide, de l'énurésie, de l'incontinence, de la déficience du facteur VIII et/ou de l'anémie à hématies falciformes, où on prépare la composition pharmaceutique stérile au moyen des étapes séparées consistant à stériliser à la chaleur la CMC, stériliser par ultrafiltration la desmopressine et combiner les composants stérilisés séparément.

9. Utilisation selon la revendication 8, où le médicament comprend une solution aqueuse de desmopressine et de CMC soluble dans l'eau.

10. Utilisation selon la revendication 8 ou 9, où la CMC est stérilisée à une température inférieure à 120°C.

11. Utilisation selon la revendication 10, où la CMC est stérilisée à une température comprise entre 105°C et 120°C.

12. Utilisation selon l une quelconque des revendications 8 à 11, où la CMC est stérilisée à la température de 105°C.

13. Utilisation selon l une quelconque des revendications 8 à 12, où on centrifuge le composant CMC stérilisée pour enlever des particules non dissoutes.

14. Utilisation selon la revendication 8 de la desmopressine en association avec de la carboxyméthylcellulose (CMC) soluble dans l eau pour la préparation d'un médicament pour le traitement de la déficience du facteur VIII.

15. Utilisation selon la revendication 14, où le médicament comprend une solution aqueuse de desmopressine et de CMC soluble dans l'eau.
